# EUROPEAN PATENT SPECIFICATION

(11) **EP 2 364 825 B1**
(45) Date of publication and mention of the grant of the patent: **18.05.2016**
(21) Application number: 11156111.4
(22) Date of filing: 25.02.2011
(51) Int. Cl.: B25J 19/02, G01L 5/22, A61B 34/30, A61B 17/00

(54) **Instrument for robotic surgery**
Instrument für die robotergestützte Chirurgie
Appareil pour la chirurgie robotique

(30) Priority: 25.02.2010 IT BO20100111
(43) Date of publication of application: 14.09.2011
(73) Proprietor: Surgica Robotica S.p.A., Trieste (IT)
(72) Inventor: Milani, Marco, 37012 Bussolengo (IT); Fiorini, Paolo, 37134 Verona (IT); Reppele, Luca, 37039 Tregnago (IT)
(74) Representative: Bergadano, Mirko

(56) References cited:
- WO-A2-03/037573
- US-A- 5 342 254
- US-B1- 6 223 100

## Description

The present invention relates to an instrument for robotic surgery.

Instruments for manual or robotic minimally invasive surgical interventions are known, in particular articulated clamps, which enable the surgeon to reach the parts concerned.

The U.S. patent No. US 6,132,441 presents a laparoscopic clamp having a jaw rigidly connected to and separate from an articulation wrist. This device envisages movements and actuations of the articulation that enable a use thereof such as to be able to grip, cut, or suture organs or tissues of a patient.

The U.S. patent No. US 7,364,582 describes an endoscopic or laparoscopic instrument, which comprises a distal member constituted, for example, by a clamp, an elongated rigid or flexible shaft for supporting the distal member, and a proximal manipulation or control member. The distal member and the proximal member are connected to the respective distal end and proximal end of the shaft via bendable movement members, and are connected together by means of a system of cables and pulleys. Said system enables bending and/or turning of the distal member, obtaining bending and/or rotation of the proximal member.

The U.S. patent No. US 6,491,701 describes an instrument for minimally invasive robotic surgery, which comprises articulated clamps that can be actuated via a system of cables and pulleys.

However, manual and robotic laparoscopic instruments, even though they are equipped with a distal articulation, reduce the tactile sensitivity of the surgeon in so far as the forces that the surgeon perceives in his hands comprise the forces of friction of the transmission system of the laparoscope, of the movement of the laparoscope in the trocar, and of the system for opening and closing the instrument. Much worse, instead, is the case of robotic surgery, in so far as current systems of robotic surgery do not envisage the reflection of force, and hence the surgeon must imagine the forces that the robot is applying on the tissues of the patient, basing his actions exclusively on the images of the operating area.

Perception of the forces is essential in so far as it enables the surgeon to perform other important actions, for example, carry out an intra-operative diagnosis on the pathological condition, prevent the use of excessive forces on tissues and organs, and finally perceive the quality of the sutures.

The patent No. US 6,233,504 describes a robot-assisted microsurgery system, which comprises a remote-control device provided with force sensors designed to detect the movements of the surgeon's hands during the operation. The movements thus detected activate a manipulator robot mechanically separate from the control device. A force-reflection element is coupled to the manipulator robot and to the control device for amplifying the tactile sensitivity of the surgeon during the operation.

Said system presents, however, the disadvantage that the force sensor is located outside the body of the patient and cannot compensate for the friction generated by the motion of the elements of the manipulator robot, such as, for example cables.

The U.S. patent application No. US 2008/0276746 illustrates a robotic-surgery system comprising a robotic arm, a terminal effector member, in particular a clamp, connected thereto in a movable way, and a force sensor set between the robotic arm and the terminal effector member.

Also said system does not, however, enable complete and effective perception of the real forces and torques in so far as the force of closing of the clamp cannot be measured by the sensor provided, and hence does not basically enable recovery of the sensitivity lost on account of the instruments.

The U.S. patent No. US 6,223,100 B1 discloses a teleoperator system for performing computer enhanced surgery with articulated instruments. The system includes right and left hand controllers for control of right and left manipulators through use of a servomechanism that includes a computer. Cameras view workspace from different angles for production of stereoscopic signal outputs at lines. In response to the camera outputs, a 3-dimensional top-to-bottom inverted image is produced which is reflected by mirror toward the eyes of operator. A virtual image is produced adjacent control arms which is viewed by operator looking in the direction of the control arms. By locating the workspace image adjacent the control arms, the operator is provided with a sense that end effectors carried by manipulator arms and control arms are substantially integral. This sense of connection between the control arms and end effectors provide the operator with the sensation of directly controlling the and effectors by hand. By locating visual display adjacent control arms image of the workspace is directly viewable by the operator. The system may employ force, torque and slip sensors of the type disclosed in U.S. patent No. 3,921,445.

The U.S. patent No. US 3,921,445 discloses a manipulator for automated machinery, including an effector, such as a hand, comprising a pair of jaws relatively pivotally movable between open and closed positions under operation of power means such as an electric motor. Sensing means, for sensing both magnitude and direction of forces along three mutually orthogonal axes intersecting at the wrist and for sensing magnitude and direction of torques about said axes, are provided at the wrist intermediate to the manipulator hand and hand supporting means. The sensing means includes a plurality of sensing units radially spaced from the longitudinal axis of the manipulator at equal distances therefrom.

The aim of the present invention is to provide an instrument for robotic surgery that will be free from the drawbacks described above; i.e., it will enable the parts concerned to be reached, as well as enabling perception of all the real forces and torques involved during the operation so as to maximize the skill of the surgeon, and at the same time will present small dimensions to enable use thereof in minimally invasive surgery, will present reliable operation, and will be easy and economically advantageous to produce. More in particular, the aim of the invention is to provide an instrument for robotic surgery that will enable detection of the possible forces of gripping when the instrument is used as a clamp.

A further aim of the present invention is to provide a device that will enable convenient change of the type of effector member that comes directly into contact with the organs of the patient.

In accordance with the present invention an instrument for robotic surgery is provided according to what is defined in the annexed claims.

The details of the invention will emerge more clearly from the detailed description of preferred embodiments of the instrument for robotic surgery according to the invention, illustrated indicatively by way of example in the annexed drawings, wherein:
- Figure 1 is a perspective view of the instrument for robotic surgery forming the subject of the invention;
- Figure 2 is a front view of the same instrument;
- Figure 3 is a side view of the same instrument;
- Figure 4 is a cross-sectional view according to the plane of trace IV-IV in Figure 2;
- Figure 5 is a cross-sectional view according to the plane of trace V-V in Figure 3;
- Figure 6 is a perspective view from beneath of a detail of the instrument according to the invention;
- Figure 7 is a front view of the same detail of the instrument forming the subject of the invention;
- Figure 8 is a cross-sectional view of the same detail according to the plane of trace VIII-VIII in Figure 7;
- Figure 9 is a perspective view of another detail of the instrument forming the subject of the invention;
- Figure 10 is a side view of a further detail of the instrument according to the invention;
- Figure 11 is a front view of the same detail;
- Figure 12 is a cross-sectional view of the same detail according to the plane of trace XII-XII in Figure 11;
- Figures 13 to 15 are, respectively, a perspective view, a plan view and a partial cross-sectional view of the detail of Figure 9 according to a further embodiment of the invention; and
- Figures 16 and 17 show respective perspective views of the further embodiment of the invention comprising the detail of Figures 13 to 15.

With reference to the above figures, designated as a whole by 1 is the instrument for robotic surgery forming the subject of the invention, which is designed to be mounted at the distal end 2 of an arm 3 of an apparatus for robotic surgery, in itself known and hence not illustrated.

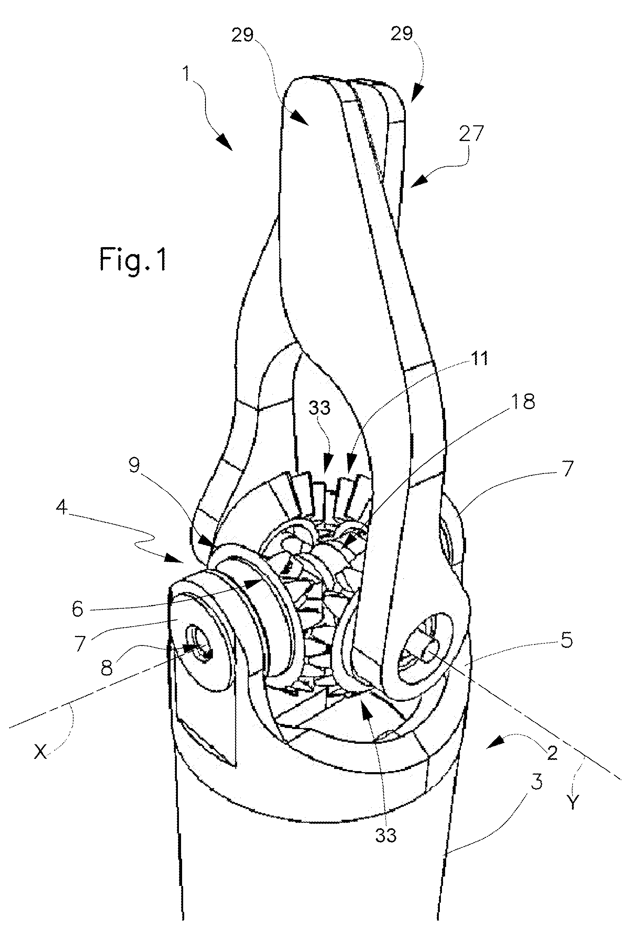

With particular reference to Figures 1 to 3, the instrument 1 comprises effector means 27 for carrying out a particular action on organs or tissues of the body of a patient during a surgical operation, and an articulation assembly 4 for supporting the effector means 27 and connecting them at the distal end 2 of the arm 3. The arm 3 has, for example, a substantially circular cross section. The articulation assembly 4 comprises a supporting ring 5 connected to the distal end 2, and transmission means 6, which can be governed, via control cables, by a movement-transmission system (not illustrated) of the apparatus for robotic surgery, the movement-transmission system being governed by the user, for transmitting movements to the effector means 27 so as to actuate them. The supporting ring 5 is longitudinally connected to the distal end 2 of the arm 3 and has an external diameter substantially equal to the external diameter of the arm 3. The supporting ring 5 comprises a pair of longitudinal arms 7, which extend in a direction orthogonal to a diameter of the supporting ring 5, are diametrally opposed and are provided, at their respective free ends, with respective through holes 8. The through holes 8 are coaxial, with the axes set preferably parallel to a diameter of the supporting ring 5.

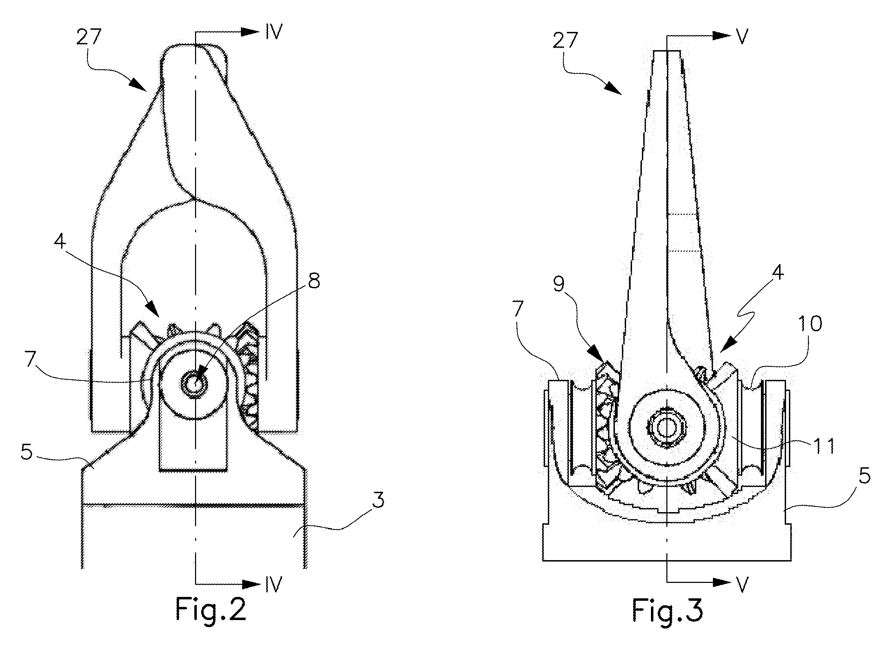

The transmission means 6 comprise a pair of bevel-gear members 9. Each bevel-gear member 9 comprises a respective pulley 10 (Figure 3), which is designed to be engaged by a respective control cable (not illustrated) of the movement-transmission system governed by the user, and a respective bevel gear 11 (Figure 3) fixed with respect to and coaxial with the pulley 10. In the example illustrated in the figures, the pulley 10 is obtained integrally in the rear part of the bevel gear 11. According to an alternative, but equivalent, embodiment, the pulley 10 and the bevel gear 11 are distinct elements appropriately connected to one another.

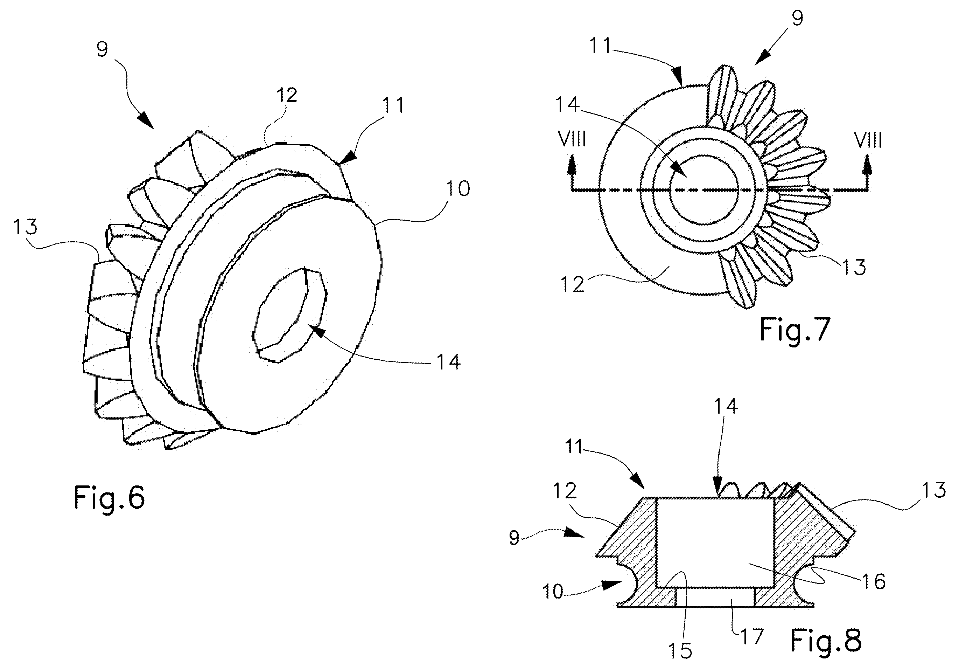

With particular reference to Figures 6 to 8, the bevel gear 11 comprises a frusto-conical outer surface having a smooth portion 12, i.e., one without teeth, and a toothed portion 13, which is complementary to the smooth portion 12 and is provided with a plurality of radially distributed teeth (Figure 7). Preferably, the teeth of the toothed portion 13 extend throughout the apothem of the frusto-conical outer surface and project perpendicularly therefrom. Each bevel-gear member 9 has an axial through hole 14, which comprises internally an annular shoulder 15 (Figure 8). The shoulder 15 defines within the axial hole 14 a first portion 16 and a second portion 17 (Figure 8). The first portion 16 extends axially between the end of the bevel gear 11 that coincides with the minor circumference of the frusto-conical outer surface and the shoulder 15. The second portion 17, which has an internal diameter smaller than that of the first portion 16, extends axially between the shoulder 15 and the side of the pulley 10 opposed to the bevel gear 11. The second portion 17 of the axial hole 14 of each bevel-gear member 9 is set substantially aligned to the respective through hole 8 of each arm 7 of the supporting ring 5.

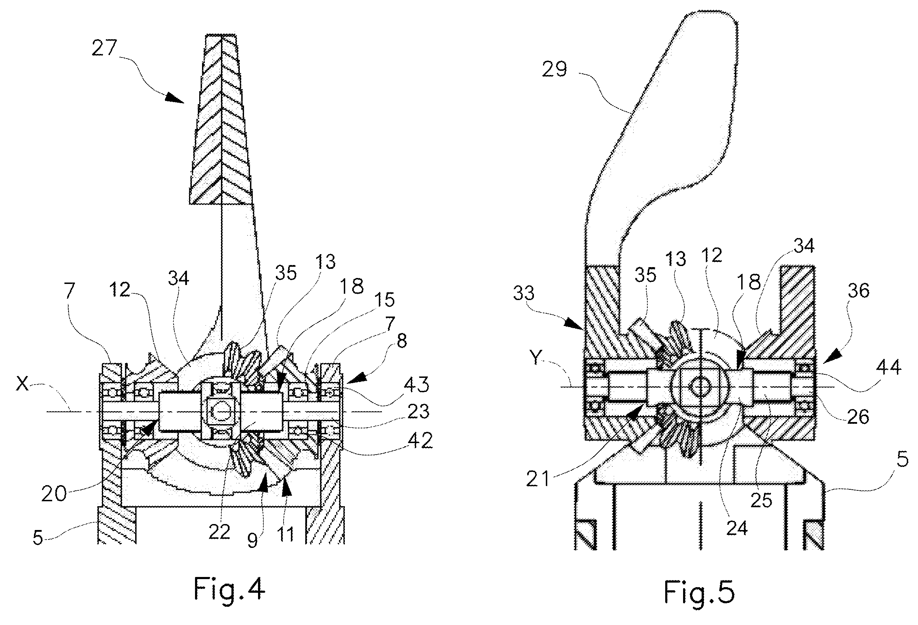

With particular reference to Figures 4, 5 and 9, the bevel-gear members 9 are mounted mobile on the arms 7 of the supporting ring 5 via a supporting and movement member 18 shaped like a cross, in particular a right cross. The supporting and movement member 18 comprises a central pulley 19 designed to be engaged by a further control cable (not illustrated) of the movement-transmission system governed by the user. Two axial arms 20 fixed with respect to the central pulley 19 extend along the axis of the central pulley 19, designated by X, from the opposite faces of the central pulley 19, and two transverse arms 21 fixed with respect to the central pulley 19 extend radially on opposite sides of the pulley 19, along an axis Y perpendicular to the axis X.

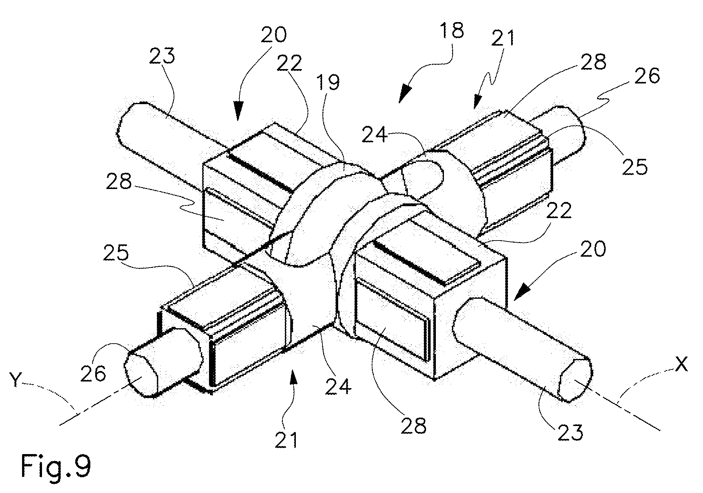

With reference to Figure 9, which illustrates the supporting and movement member 18 removed from the instrument 1, each axial arm 20 comprises a detection portion 22 adjacent to the pulley 19 and having a preferably polygonal cross section, for example square, and a substantially cylindrical coupling portion 23. The detection portion 22 has a maximum transverse encumbrance substantially corresponding to the external diameter of the central pulley 19. The coupling portion 23 has, instead, a cross section having an extension smaller than that of the cross section of the detection portion 22. Moreover, the distance between the corresponding outermost ends of the axial arms 20 is substantially equal to the external diameter of the supporting ring 5.

The coupling portion 23 of each axial arm 20 is rotatably inserted both in the axial hole 14 of a respective bevel-gear member 9 and in the through hole 8 of a respective longitudinal arm 7 of the supporting ring 5 (Figure 4). In this way, the supporting and movement member 18 rotatably supports the bevel-gear members 9 and is rotatably supported by the supporting ring 5. The portion 16 of the axial hole 14 of each bevel-gear member 9 has a diameter greater than that of the central pulley 19 and a depth such as to house at least one longitudinal portion of the corresponding detection portion 22 (Figure 4).

Each transverse arm 21 comprises a proximal portion 24, which is fixed with respect to the central pulley 19, has a preferably circular cross section, and is appropriately equipped with a passage for the cables for controlling the central pulley 19. Extending coaxially from this proximal portion 24 of the transverse arm 21 is a transverse detection portion 25 oriented radially with respect to the central pulley 19 and having a preferably polygonal cross section, for example square. The transverse encumbrance of the transverse detection portion 25 corresponds substantially to the external diameter of the proximal portion 24 of the transverse arm 21. Extending coaxially from the transverse detection portion 25 is a transverse coupling portion 26 with circular cross section. The transverse coupling portion 26 preferably has a transverse encumbrance smaller than that of the transverse detection portion 25 of the transverse arm 21 itself. The transverse coupling portions 26 of the transverse arms 21 are rotatably connected to the effector means 27 (Figures 1 to 4) in order to obtain the desired effects in the body of the patient, as explained more fully hereinafter.

The detection portion 22 of the axial arms 20 and the transverse detection portion 25 of the transverse arms 21 have respective longitudinal plane faces substantially parallel or respectively perpendicular to the plane in which the axes X and Y of the axial arm 20 and transverse arm 21, respectively, of the supporting and movement member 18 lie. Set on the faces of each of the detection portions 22 and 25 are force sensors 28 for detecting the forces applied to the supporting and movement member 18. The cross-like conformation of the supporting and movement member 18 enables the force sensors 28 to detect the three forces and the three torques that are, respectively, directed along and rotating about the cartesian axes and that are applied to the effector means 27 by an external dynamic load. In particular, each force sensor 28 is constituted, for example, by a strain gauge. When the cables around the pulleys 10 and 19 are actuated by the movement-transmission system, the axial arms 20 and the transverse arms 21 undergo respective bending that is detected by the force sensors 28.

The effector means 27 preferably comprise two jaws 29 (Figure 5), each of which is rotatably mounted on a transverse connection portion 26 of a respective transverse arm 21 of the supporting and movement member 18.

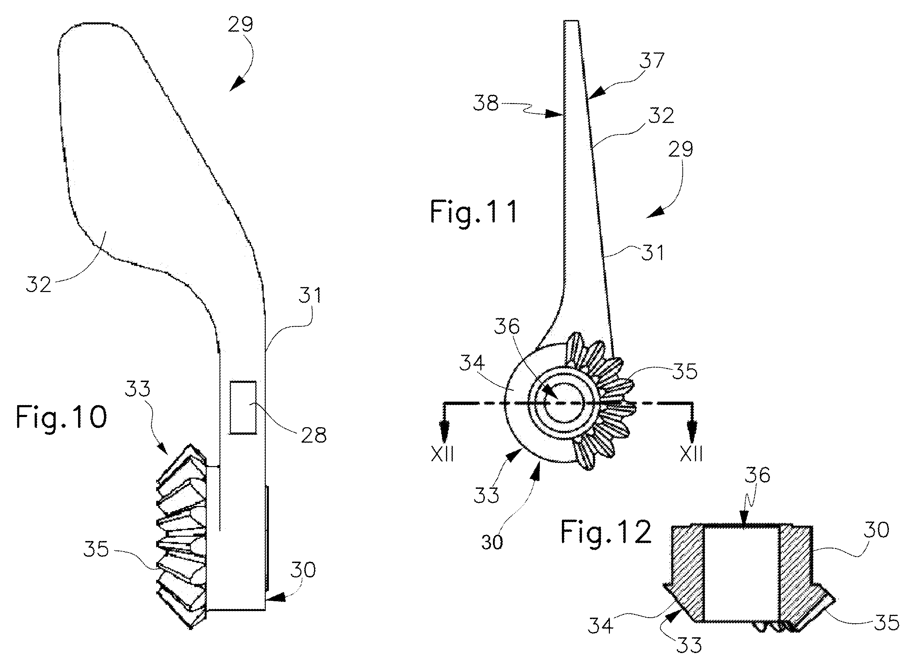

With particular reference to Figures 10 to 12, each jaw 29 comprises a connection base 30, a supporting arm 31, and a terminal member 32. The connection base 30, which is preferably circular in shape, comprises a bevel gear 33 that projects coaxial from the connection base 30 itself. In the example illustrated in Figure 10, the connection base 30 and the bevel gear 33 are made of a single piece. According to an alternative embodiment, the connection base 30 and the bevel gear 33 are made as distinct elements and are connected together. The bevel gear 33 comprises a frusto-conical outer surface having a smooth portion 34, i.e., without teeth, and a toothed portion 35, which is complementary to the smooth portion 34 and is provided with a plurality of teeth similar to the teeth of the toothed portion 13 of the bevel-gear members 9. The connection base 30 further comprises a centring hole 36, which is rotatably engaged by the transverse connection portion 26 of a transverse arm 21 of the supporting and movement member 18. The supporting arm 31 projects from the connection base 30 in a substantially coplanar way. Preferably, the supporting arm 31 develops extending from the connection base 30 in a position corresponding to the toothed portion 35 of the bevel gear 33. The terminal member 32 set at the end of the supporting arm 31 is preferably set according to a plane comprising the axis of the centring hole 36 of the connection base 30 and has a curved shape oriented on the side of the bevel gear 33 so as to be able to have effect in a plane containing the longitudinal axis of the arm 3 of the apparatus for robotic surgery.

The transverse coupling portion 26 of each of the transverse arms 21 of the supporting and movement member 18 is rotatably inserted in the centring hole 36 of the connection base 30 of a respective jaw 29. In this way, the transverse arms 21 rotatably support the effector means 27. The centring hole 36 of each jaw 29 has a diameter greater than that of the proximal portion 24 of a respective transverse arm 21 and a depth such as to house the transverse detection portion 25 and at least one longitudinal portion of the proximal portion 24 (Figure 5).

In the example of embodiment illustrated in the figures, the two jaws 29 of the effector means 27 work as a clamp, moving from a closing configuration, in which desired parts are gripped, and an opening configuration, in which the desired parts are released. For said purpose, each terminal member 32 preferably has a flattened and curved shape as illustrated in Figures 1 to 5, 10 and 11 and described previously. Each terminal member 32 has a free outer face 37, which coincides, for example, with a plane tangential to the connection base 30 of the jaw 29, and an inner operative face 38, which preferably coincides with a plane containing the axis of the connection base 30 and the longitudinal axis of the arm 3 (see, in particular, Figure 11). In practice, in a condition of closing of the jaws 29, the respective operative faces 38 are set against one another, whilst in an opening condition the operative faces 38 are oriented in a divergent way, set apart from of one another.

Preferably set internally, externally, or on both sides of the supporting arms 31 of the jaws 29 are further force-sensor means 28 for detecting the force of closing of the effector means 27.

According to further embodiments (not illustrated), the effector means 27 are made in the form of scissors or in the form of another type of surgical instrument having a different function, but comprising, in any case, a single jaw or a pair of jaws that move towards one another or away from one another according to the operation to be performed. It should be noted that the connection base 30, the supporting arm 31, and the terminal member 32 of a single jaw 29 are preferably made as a single component. Hence, by simple replacement of just the jaws 29 it is possible to change the type of the effector means 27, for example from gripping means to cutting means.

With reference once again to Figure 4, the coupling portion 23 of each axial arm 20 of the supporting and movement member 18 is inserted in the axial hole 14 of the respective bevel-gear member 9 by interposition of a respective rolling supporting member 42. The rolling supporting members 42 are mounted against the annular shoulder 15 of the axial hole 14. Furthermore, the end of the coupling portion 23 of each axial arm 20 is inserted in the through hole 8 of the respective longitudinal arm 7 of the supporting ring 5 by interposition of a further respective rolling supporting member 43. The rolling supporting members 42 and 43 are constituted, for example, by respective ball bearings. In this way, the bevel-gear members 9 are free to turn about the axis X both with respect to the supporting and movement member 18 and with respect to the longitudinal arms 7 of the supporting ring 5.

With reference once again to Figure 5, the transverse coupling portion 26 of each of the transverse arms 21 of the supporting and movement member 18 is inserted in the centring hole 36 of the connection base 30 of a respective jaw 29 by interposition of a respective rolling supporting member 44. The rolling supporting members 44 are constituted, for example, by respective ball bearings. In this way, the jaws 29 are free to turn about the axis Y with respect to the supporting and movement member 18.

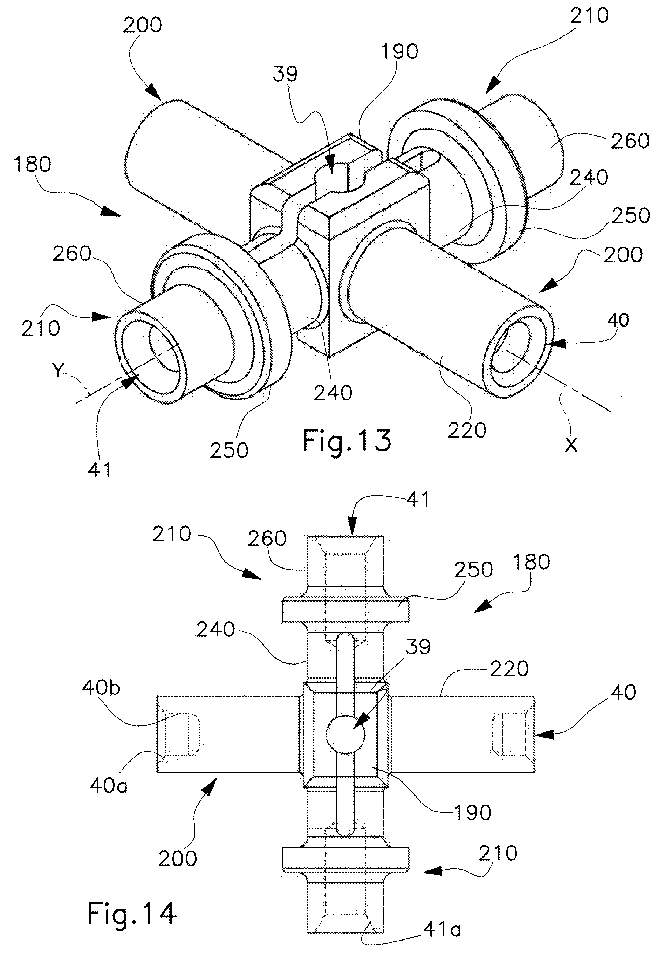

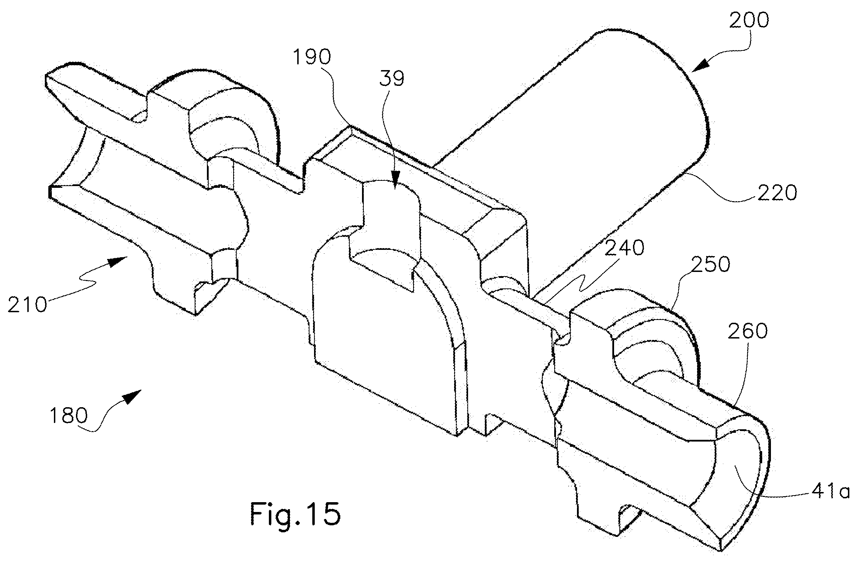

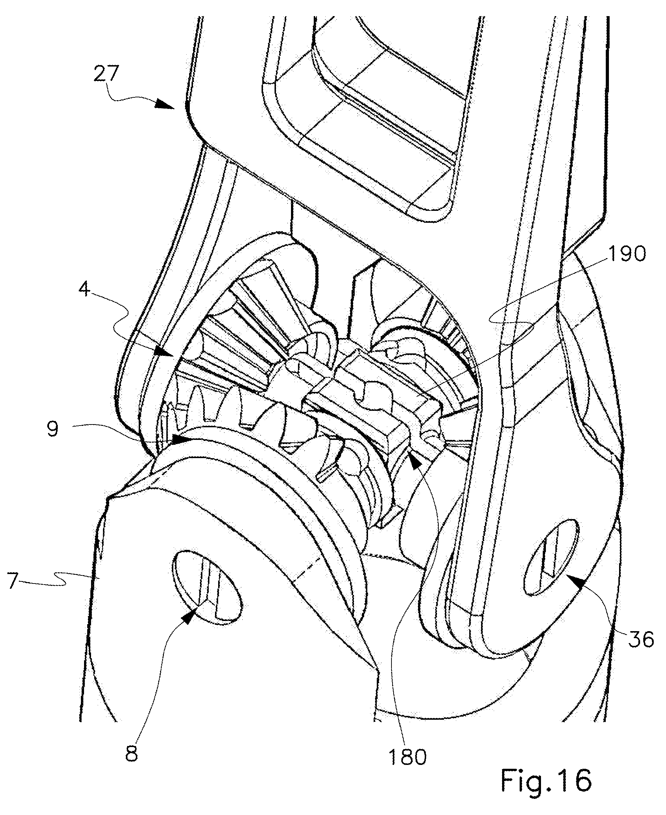

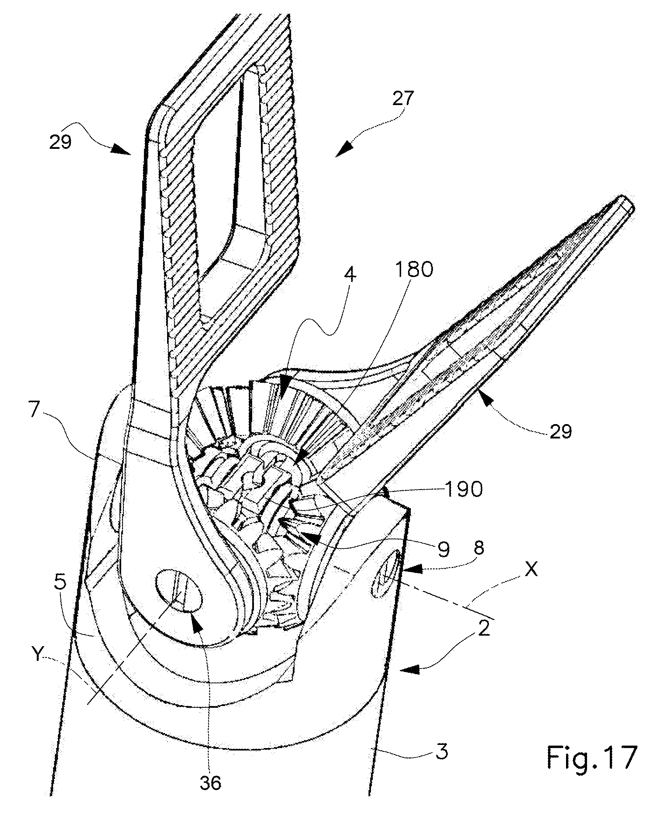

According to a further embodiment of the invention illustrated in Figures 13 to 17, the central pulley 190 of the supporting and movement member 180 externally has a parallelepipedal shape. The central pulley 190 comprises a transverse groove 39 set in a substantially median position between a pair of parallel faces of the aforesaid central parallelepipedal pulley 190. More precisely, the transverse groove 39 is designed to define a channel of passage such as a crimping seat for winding and/or connection of a respective cable (not illustrated). As in the case of the embodiment illustrated in Figures 1 to 12, the supporting and movement member 180 comprises two axial arms 200, which extend along the axis X, and two transverse arms 210, which project in a cross from respective lateral faces of the central pulley 190, i.e., which extend along the axis Y orthogonal to the axis X. The axial arms 200 each comprise a connection portion 220 cylindrical in shape provided at the respective free end of an axial blind hole 40 with circular cross section. Each axial hole 40 preferably comprises an inlet area 40a of a frusto-conical shape with cross section decreasing from the edge inwards, connected to a cylindrical connection portion 40b. The connection portion 40b of the axial hole 40 is preferably threaded to enable connection to the longitudinal arms 7 via purposely provided screw means (Figures 16 and 17).

Each transverse arm 210 comprises a respective proximal portion 240, which is preferably cylindrical and extends from a respective lateral face of the central pulley 190, and an intermediate portion 250, which has a circular cross section, is coaxial to the proximal portion 240, and extends from the end of the proximal portion 240 itself. Preferably, the intermediate portion 250 has an axial and radial extension such as to enable insertion thereof, preferably in hide-away fashion, in the aforesaid centring hole 36 of a corresponding bevel gear 33 associated to one of the jaws 29. Each transverse arm 210 comprises a transverse coupling portion 260, which extends coaxially from the intermediate portion 250 and is inserted in the centring hole 36 itself. Each coupling portion 260 has, at its free end, a respective axial opening 41, which comprises a preferably tapered internal edge 41a for facilitating insertion of fixing means, constituted, for example, by a screw. The axial opening 41 extends longitudinally as far as the proximal portion 240, inside which it is in communication with the transverse groove 39 to enable passage of the cable.

The bevel-gear members 9 are rotatably mounted on the connection portions 220 of the axial arms 200 of the supporting and movement member 180 so as to be set between the central pulley 190 and the longitudinal arms 7 of the supporting ring 5. The ends of the axial arms 200 are inserted in the through holes 8 of the longitudinal arms 7 of the supporting ring 5 by interposition of rolling supporting members and fixed axially for example by screw means that engage the axial holes 40.

The jaws 29 of the effector means 27 are rotatably mounted on the transverse connection portions 260 of the transverse arms 210 of the supporting and movement member 180 and are blocked, in the direction of installation parallel to the axis of the transverse arms 210, so as to bear upon the intermediate portions 250 via fixing members, for example screw means, which engage the axial opening 41 (Figures 16 and 17).

Operation of the instrument for robotic surgery forming the subject of the invention may, in both of the embodiments described, be readily understood from the foregoing description and is, however, described hereinafter for greater clarity.

Each bevel-gear member 9 has the bevel gear 11 set with the toothed portion 13 meshing with the toothed portion 35 of the bevel gear 33 of one jaw 29 and with the smooth portion 12 substantially facing the smooth portion 34 of the bevel gear 33 of the other jaw 29. In this way, each bevel-gear member 9 is kinematically coupled only to a respective one of the jaws 29; i.e., the bevel gear 11 of each bevel-gear member 9 meshes only with the bevel gear 33 of a respective jaw 29, and vice versa. This enables, in use, movement the two jaws 29 independently of one another.

In fact, the cables that engage the pulleys 10 of the bevel-gear members 9 enable, in use, independent rotations, about the axis X, to be imparted on the bevel-gear members 9, each of which then transmits a corresponding movement of rotation only to a respective jaw 29, which turns about the axis Y. According to the direction and amplitude of the angle of rotation of the bevel-gear members 9 it is possible to close or open the jaws 29 or else turn the effector means 27 about the axis Y, without affecting the state of opening or closing of the jaws 29. For instance, by turning the two bevel-gear members 9 in opposite directions according to two equal angles of opening, opening or closing of the jaws 29 is obtained. Instead, by turning the two bevel-gear members 9 again according to two equal angles of opening but in the same direction, rotation of both of the jaws 29 about the axis Y is obtained without affecting their state of opening or closing.

Instead, the cable that engages the central pulley 19 enables, in use, a rotation of the entire supporting and movement member 18, 180, and of the effector means 27 mounted on it, about the axis X, to be imparted, thus causing rotation of the effector means 27 about the axis X without affecting the state of opening or closing of the jaws 29.

The instrument for robotic surgery achieves the purpose of enabling the parts concerned to be reached and enables perception of all the real forces and torques involved during a surgical operation so as to maximize the skill and sensitivity of the surgeon. Said purpose is achieved, in particular, thanks to the articulation assembly 4 and the sensor means 28 associated thereto.

The supporting and movement member 18, 180 of the articulation assembly 4 in fact enables movement in an optimal way of the effector means 27, whilst the sensor means 28 are set in such a way as to detect forces and torques that are not affected by the forces of friction on the components of the surgical instrument 1. In particular, the force sensors 28 set on the supporting arms 31 of the jaws 29 enable effective detection of the force of gripping exerted by the effector means 27 when they are used as clamp.

One advantage of the instrument forming the subject of the invention is constituted by the cross-like conformation of the supporting and movement member 18, 180, which bestows on the surgical instrument a high structural compactness and ease of use, in addition to rendering it particularly suitable for use in minimally invasive surgery. Said constructional solution moreover represents as a whole an optimal approximation of a spherical wrist. Unlike instruments of a known type, this enables, among other things, rotation of the effector means 27 about two mutually perpendicular axes with just one articulation assembly.

Furthermore, by means of the arrangement of suitable force-sensor means 28 on the respective axial and transverse arms of the supporting and transmission member 18, 180 it is possible to detect all the forces and torques applied by an external load on the surgical instrument 1.

A prerogative of the instrument for robotic surgery forming the subject of the invention is represented by the fact that the articulation assembly 4 comprises the bevel gears 11, 33 mounted on the supporting and movement member 18, 180. Said gears enable easy and independent movement of each supporting arm 31 of the effector means 27.

One advantage of the instrument forming the subject of the invention is represented by the fact that the effector means 27 can be easily replaced, being rotatably supported by the transverse arms 21, 210 of the supporting and movement member 18, 180 and mounted by interposition of rolling supporting members.

Finally, it should be noted that the instrument forming the subject of the invention is built in a simple and sturdy way, in addition to having a contained cost, adapted for the requirements of a consumer product. In the practical embodiment of the invention, the materials used, as well as the shape and the dimensions, can be any according to the needs.

Where the technical characteristics mentioned in each claim are followed by reference signs, said reference signs have been included merely to facilitate understanding of the claims and consequently do not have any value that might limit the purpose of each element identified, by way of example, by said reference signs.

## Claims

1. An instrument for robotic surgery designed to be mounted at the distal end (2) of an operative arm (3) of an apparatus for robotic surgery, the instrument (1) comprising: effector means (27) for performing a given action on the body of a patient during a surgical operation; an articulation assembly (4) for supporting said effector means (27) and connecting the effector means (27) to said distal end (2) of said operative arm (3); and first sensor means (28), which are associated to said articulation assembly (4) for detecting forces and torques applied to said effector means (27); the instrument (1) **being characterized in that** said articulation assembly (4) comprises: a supporting and movement member (18; 180), which is cross-shaped, is articulated to said distal end (2) of said operative arm (3), and is designed to support said effector means (27) in a mobile way; and transmission means (6), which are mounted on said supporting and movement member (18; 180) and are kinematically coupled to said effector means (27) and are configured to engage with control cables of said apparatus for robotic surgery for actuating said effector means (27).

2. The instrument for robotic surgery according to Claim 1, **characterized in that** said first sensor means (28) are mounted on said supporting and movement member (18; 180).

3. The instrument for robotic surgery according to Claim 1 or 2, **characterized in that** it comprises second sensor means (28) set on said effector means (27) for detecting forces internal to said effector means (27).

4. The instrument for robotic surgery according to any one of Claims 1 to 3, **characterized in that** said articulation assembly (4) comprises a supporting ring (5) connected to said distal end (2) of said operative arm (3) for rotatably supporting said supporting and movement member (18, 180).

5. The instrument for robotic surgery according to Claim 4, **characterized in that** said supporting and movement member (18; 180) comprises a central pulley (19; 190) designed to be engaged by a control cable of said apparatus for robotic surgery, two axial arms (20; 200), which are fixed with respect to the central pulley (19; 190) and extend along a first axis (X) coinciding with the axis of the central pulley (19; 190), and two transverse arms (21; 210), which are fixed with respect to the central pulley (19; 190) and extend radially on opposite sides of the central pulley (19; 190) along a second axis (Y) orthogonal to the first axis (X) for supporting said effector means (27) rotatably about the second axis (Y); said supporting ring (5) rotatably supporting said axial arms (20; 200) in such a way that said supporting and movement member (18; 180) and said effector means (27) can turn together about the first axis (X) according to the commands imparted by said control cable.

6. The instrument for robotic surgery according to Claim 5, **characterized in that** said transmission means (6) comprise bevel-gear members (9) rotatably mounted on said axial arms (20; 200) of said supporting and movement member (18; 180) to turn about said first axis (X).

7. The instrument for robotic surgery according to Claim 6, **characterized in that** said bevel-gear members (9) comprise at least one further pulley (10), which is designed to be engaged by a respective further control cable of said apparatus for robotic surgery, and at least one first bevel gear (11) associated to said further pulley (10) for transmitting the movements imparted by the further control cables to said effector means (27).

8. The instrument for robotic surgery according to Claim 7, **characterized in that** said effector means (27) comprise at least one jaw (29), which is rotatably mounted on one of said transverse arms (21; 210) to turn about said second axis (Y) and comprises a respective second bevel gear (33) meshing with said first bevel gear (11) of said bevel-gear members (9).

9. The instrument for robotic surgery according to Claim 8, **characterized in that** said first bevel gear (11) comprises a first frusto-conical outer surface having a first smooth portion (12) and a first toothed portion (13), which is complementary to the first smooth portion (12), and said second bevel gear (33) comprises a second frusto-conical outer surface having a second smooth portion (34) and a second toothed portion (35), which is complementary to the second smooth portion (34); the first bevel gear (11) being set with the first toothed portion (13) meshing with the second toothed portion (35) of said second bevel gear (33).

10. The instrument for robotic surgery according to Claim 5, **characterized in that** said transmission means (6) comprise two bevel-gear members (9), each of which is rotatably mounted on a respective one of said axial arms (20; 200) to turn about said first axis (X), and said effector means (27) comprise two jaws (29), each of which is rotatably mounted on a respective one of said transverse arms (21; 210) to turn about said second axis (Y) and is kinematically coupled only to a respective one of said bevel-gear members (9) to enable actuation of the two jaws (29) independently of one another.

11. The instrument for robotic surgery according to Claim 10, **characterized in that** each of said bevel-gear members (9) comprises a respective further pulley (10), which is designed to be engaged by a respective further control cable of said apparatus for robotic surgery, and at least one respective first bevel gear (11) associated to said further pulley (10); each jaw (29) comprising a respective second bevel gear (33), which meshes with the first bevel gear (11) of the bevel-gear member (9) associated to the jaw (29) for transmitting the movements imparted by the respective further control cable to the corresponding jaw (29).

12. The instrument for robotic surgery according to Claim 11, **characterized in that** each said first bevel gear (11) comprises a respective first frusto-conical outer surface having a first smooth portion (12) and a first toothed portion (13), which is complementary to the first smooth portion (12), and each said second bevel gear (33) comprises a respective second frusto-conical outer surface having a second smooth portion (34) and a second toothed portion (35), which is complementary to the second smooth portion (34); the first bevel gear (11) of each bevel-gear member (9) being set with the first toothed portion (13) meshing with the second toothed portion (35) of the second bevel gear (33) of one jaw (29) and with the first smooth portion (12) substantially facing the second smooth portion (34) of the second bevel gear (33) of the other jaw (29).

13. The instrument for robotic surgery according to Claim 5, **characterized in that** each of said axial and transverse arms (20, 21) of said supporting and movement member (18; 180) comprises a respective detection portion (22, 25), which has at least one plane-surface portion parallel or orthogonal to a plane in which said first and second axes (X, Y) lie; said first sensor means (28) being set on at least one of the plane-surface portions.

14. The instrument for robotic surgery according to Claim 3, **characterized in that** said effector means (27) comprise at least one jaw (29), which is rotatably mounted on said supporting and movement member (18, 180) and comprises a supporting arm (31) and a terminal member (32); said second sensor means (28) being set on at least one side of said supporting arm (31).

## Patentansprüche

1. Instrument für eine Roboteroperation, welches dafür vorgesehen ist, am distalen Ende (2) eines Betätigungsarms (3) eines Apparates für Roboteroperationen angeordnet zu werden, mit:
Effektormitteln (27) zur Ausführung einer bestimmten Tätigkeit an einem Patientenkörper während einer chirurgischen Operation; einer Gelenkanordnung (4), die die Effektormittel (27) unterstützt und die Effektormittel (27) mit dem distalen Ende (2) des Betätigungsarms (3) verbindet; und mit ersten Erfassungsmitteln (28), die der Gelenkanordnung (4) zur Erfassung von auf das Effektormittel (27) ausgebübten Kräften und Momenten zugeordnet sind, **dadurch gekennzeichnet, dass** die Gelenkanordnung (4) Folgendes aufweist: ein Stütz- und Bewegungsglied (18; 180), das kreuzförmig ausgebildet und an dem distalen Ende (2) des Betätigungsarms (3) angelenkt ist und dazu vorgesehen ist, das Effektormittel (27) in beweglicher Art und Weise zu unterstützen; und mit Übertragungsmitteln (6), welche an dem Stütz- und Bewegungsglied (18; 180) angeordnet und
mit den Effektormitteln (27) kinematisch gekoppelt sind und dazu eingerichtet sind, mit Steuerkabeln des Apparates für Roboteroperationen in Eingriff zu stehen, um die Effektormittel (27) zu betätigen.

2. Das Instrument für eine Roboteroperation nach Anspruch 1, **dadurch gekennzeichnet, dass** die ersten Erfassungsmittel (28) an dem Stütz- und Bewegungsglied (18; 180) angeordnet sind.

3. Das Instrument für eine Roboteroperation nach Anspruch 1 oder 2, **dadurch gekennzeichnet, dass** es ein zweites Erfassungsmittel (28) an den Effektormitteln (27) aufweist, um innere Kräften an den Effektormitteln (27) zu erfassen.

4. Das Instrument für eine Roboteroperation nach einem der Ansprüche 1 bis 3, **dadurch gekennzeichnet, dass** die Gelenkanordnung (4) einen Stützring (5) aufweist, welcher mit dem distalen Ende (2) des Betätigungsarms (3) verbunden ist, um das Stütz- und Bewegungsglied (18; 180) drehbar zu stützen.

5. Das Instrument für eine Roboteroperation nach Anspruch 1, **dadurch gekennzeichnet, dass** das Stütz- und Bewegungsglied (18; 180) eine zentrale Scheibe (19; 190) aufweist, die dazu vorgesehen ist, mit einem Steuerkabel des Apparates zur Roboteroperation in Eingriff zu stehen, mit zwei Axialarmen (20; 200), die bezüglich der zentralen Scheibe (19; 190) festgelegt sind und sich entlang einer ersten Achse (X) erstrecken, die mit der Achse der zentralen Scheibe (19; 190) zusammenfällt, und mit zwei Querarmen (21; 210), die bezüglich der zentralen Scheibe (19; 190) festgelegt sind und sich radial an gegenüberliegenden Seiten der zentralen Scheibe (19; 190) entlang einer zweiten, senkrecht zur ersten Achse (X) stehenden Achse (Y) erstrecken, um das Effektormittel (27) drehbar um die zweite Achse (Y) zu stützen, wobei der Stützring (5) die Axialarme (20; 200) drehbar in einer Art und Weise stützt, dass das Stütz- und Bewegungsglied (18; 180) und die Effektormittel (27) sich entsprechend der von den Kontrollkabeln vermittelten Befehle zusammen um die erste Achse (X) drehen können.

6. Das Instrument für eine Roboteroperation nach Anspruch 5, **dadurch gekennzeichnet, dass** die Übertragungsmittel (6) Kegelradglieder (9) aufweisen, die drehbar an den Axialarmen (20; 200) des Stütz- und Bewegungsglieds (18; 180) angeordnet sind, um sich um die erste Achse (X) zu drehen.

7. Das Instrument für eine Roboteroperation nach Anspruch 5, **dadurch gekennzeichnet, dass** die Kegelradglieder (9) zumindest eine weitere Scheibe (10) aufweisen, die dazu eingerichtet ist, mit einem weiteren Steuerkabel des Apparates für Roboteroperationen in Eingriff zu stehen und mit wenigstens einem weiteren ersten Kegelrad (11), welches der weiteren Scheibe zum Übermitteln der durch das weitere Steuerkabel an die Effektormittel (27) vermittelten Bewegungen zugeordnet ist.

8. Das Instrument für eine Roboteroperation nach Anspruch 7, **dadurch gekennzeichnet, dass** die Effektormittel (27) mindestens eine an einem der Querarme (21; 210) drehbar angeordnete Klaue (29) aufweisen, die um die zweite Achse (Y) drehbar ist und mit einem entsprechenden zweiten Kegelrad (33) versehen ist, das mit dem ersten Kegelrad (11) der Kegelradglieder (9) kämmt.

9. Das Instrument für eine Roboteroperation nach Anspruch 8, **dadurch gekennzeichnet, dass** das erste Kegelrad (11) eine erste kegelstrumpfförmige äußere Oberfläche mit einem ersten ebenen Abschnitt (12) und einem ersten gezahnten Abschnitt (13) umfasst, der den ersten ebenen Abschnitt ergänzt, und dass das zweite Kegelrad (33) eine zweite kegelstumpfförmige äußere Oberfläche mit einem zweiten ebenen Abschnitt (34) und einem zweiten gezahnten Abschnitt (35) umfasst, der den zweiten ebenen Abschnitt (34) ergänzt, wobei das erste Kegelrad (11) mit dem ersten gezahnten Abschnitt (13) so eingerichtet ist, dass dieser mit dem zweiten gezahnten Abschnitt (35) des zweiten Kegelrads (33) kämmt.

10. Das Instrument für eine Roboteroperation nach Anspruch 5, **dadurch gekennzeichnet, dass** das Übertragungsmittel (6) zwei Kegelradglieder (9) aufweist, von denen jedes drehbar an dem jeweiligen Axialarm (20; 200) zur Drehung um die erste Achse (X) angeordnet ist, und dass die Effektormittel (27) zwei Klauen (29) umfassen, von denen jede drehbar an dem jeweiligen Querarm zur Drehung um die zweite Achse (Y) angeordnet und kinematisch lediglich mit einem entsprechenden der Kegelradglieder gekoppelt ist, um eine von der jeweils anderen unabhängige Betätigung der beiden Klauen (29) zu ermöglichen.

11. Das Instrument für eine Roboteroperation nach Anspruch 10, **dadurch gekennzeichnet, dass** jedes der Kegelradglieder (9) jeweils eine weitere Scheibe (10) umfasst, die dazu eingerichtet ist, mit einem entsprechenden weiteren Steuerkabel des Apparats zur Roboteroperation in Eingriff zu stehen, und mit jeweils wenigstens einem den weiteren Scheiben (10) zugeordneten ersten Kegelrad (11), wobei jede Klaue (29) jeweils ein zweites Kegelrad (33) aufweist, das mit dem ersten Kegelrad (11) des der Klaue zugeordneten Kegelradglieds (9) kämmt, um die von dem jeweiligen weiteren Steuerkabel an die entsprechende Klaue (29) vermittelten Bewegungen zu übermitteln.

12. Das Instrument für eine Roboteroperation nach Anspruch 10, **dadurch gekennzeichnet, dass** jedes erste Kegelrad (11) jeweils eine erste kegelstrumpfförmige äußere Oberfläche mit einem ersten ebenen Abschnitt (12) und einem ersten gezahnten Abschnitt (13) umfasst, der den ersten ebenen Abschnitt (12) ergänzt, und dass das zweite Kegelrad (33) eine zweite kegelstumpfförmige äußere Oberfläche mit einem zweiten ebenen Abschnitt (34) und einem zweiten gezahnten Abschnitt (35) umfasst, der den zweiten ebenen Abschnitt (34) ergänzt, wobei das erste Kegelrad (11) jedes Kegelradglieds (9) derart eingerichtet ist, dass der erste gezahnte Abschnitt (13) mit dem zweiten gezahnten Abschnitt (35) des zweiten Kegelrads (33) einer Klaue (29) kämmt, während ein erster ebener Abschnitt (12) im Wesentlichen dem zweiten ebenen Abschnitt (34) des zweiten Kegelrads (33) einer anderen Klaue (29) gegenübersteht.

13. Das Instrument für eine Roboteroperation nach Anspruch 5, **dadurch gekennzeichnet, dass** jeder der Axial- und Querarme (20, 21) des Stütz- und Bewegungsglieds (18; 180) jeweils einen Erfassungsabschnitt (22, 25) aufweist, der wenigstens einen ebenen Flächenabschnitt parallel oder senkrecht zu derjenigen Fläche aufweist, in der die ersten und zweiten Achsen (X, Y) liegen; wobei die ersten Erfassungsmittel (28) an wenigstens einem der ebenen Flächenabschnitte angeordnet sind.

14. Das Instrument für eine Roboteroperation nach Anspruch 3, **dadurch gekennzeichnet, dass** die Effektormittel (27) wenigstens jeweils eine Klaue (29) aufweisen, die drehbar an dem Stütz- und Bewegungsglied (18; 180) angeordnet ist, und einen Stützarm (31) und ein Endglied (32) aufweist, wobei das zweite Erfassungsmittel (28) an wenigstens einer Seite des Stützarms (31) angeordnet ist.

## Revendications

1. Instrument pour la chirurgie robotique conçue pour être monté sur l'extrémité distale (2) d'un bras opérationnel (3) d'un appareil pour la chirurgie robotique, l'instrument (1) comprenant : des moyens d'effecteur (27) pour réaliser une action donnée sur le corps d'un patient pendant une opération chirurgicale ; un ensemble d'articulation (4) pour supporter lesdits moyens d'effecteur (27) et raccorder les moyens d'effecteur (27) à ladite extrémité distale (2) dudit bras opérationnel (3) ; et des premiers moyens de capteur (28) qui sont associés audit ensemble d'articulation (4) pour détecter des forces et des couples appliqués auxdits moyens d'effecteur (27) ; l'instrument (1) étant **caractérisé en ce que** ledit ensemble d'articulation (4) comprend : un élément de support et de déplacement (18 ; 180) qui est en forme de croix, est articulé par rapport à ladite extrémité distale (2) dudit bras opérationnel (3) et est conçu pour supporter lesdits moyens d'effecteur (27) d'une manière mobile ; et des moyens de transmission (6) qui sont montés sur ledit élément de support et de déplacement (18 ; 180) et sont couplés de manière cinématique auxdits moyens d'effecteur (27) et sont configurés pour se mettre en prise avec des câbles de commande dudit appareil pour la chirurgie robotique afin d'actionner lesdits moyens d'effecteur (27).

2. Instrument pour la chirurgie robotique selon la revendication 1, **caractérisé en ce que** lesdits premiers moyens de capteur (28) sont montés sur ledit élément de support et de déplacement (18 ; 180).

3. Instrument pour la chirurgie robotique selon la revendication 1 ou 2, **caractérisé en ce qu'**il comprend des seconds moyens de capteur (28) placés sur lesdits moyens d'effecteur (27) pour détecter des forces à l'intérieur desdits moyens d'effecteur (27).

4. Instrument pour la chirurgie robotique selon l'une quelconque des revendications 1 à 3, **caractérisé en ce que** ledit ensemble d'articulation (4) comprend une bague de support (5) raccordée à ladite extrémité distale (2) dudit bras opérationnel (3) pour supporter en rotation ledit élément de support et de déplacement (18, 180).

5. Instrument pour la chirurgie robotique selon la revendication 4, **caractérisé en ce que** ledit élément de support et de déplacement (18 ; 180) comprend une poulie centrale (19 ; 190) conçue pour être mise en prise par un câble de commande dudit appareil pour la chirurgie robotique, deux bras axiaux (20 ; 200) qui sont fixes par rapport à la poulie centrale (19 ; 190) et s'étendent le long d'un premier axe (X) coïncidant avec l'axe de la poulie centrale (19 ; 190) et deux bras transversaux (21 ; 210) qui sont fixes par rapport à la poulie centrale (19 ; 190) et s'étendent radialement sur les côtés opposés de la poulie centrale (19 ; 190) le long d'un second axe (Y) orthogonal au premier axe (X) pour supporter lesdits moyens d'effecteur (27) en rotation autour du second axe (Y) ; ladite bague de support (5) supportant en rotation lesdits bras axiaux (20 ; 200) de sorte que ledit élément de support et de déplacement (18 ; 180) et lesdits moyens d'effecteur (27) peuvent tourner ensemble autour du premier axe (X) selon les commandes communiquées par ledit câble de commande.

6. Instrument pour la chirurgie robotique selon la revendication 5, **caractérisé en ce que** lesdits moyens de transmission (6) comprennent des éléments de pignon conique (9) montés en rotation sur lesdits bras axiaux (20 ; 200) dudit élément de support et de déplacement (18 ; 180) afin de tourner autour dudit premier axe (X).

7. Instrument pour la chirurgie robotique selon la revendication 6, **caractérisé en ce que** lesdits éléments de pignon conique (9) comprennent au moins une autre poulie (10) qui est conçue pour être mise en prise par un autre câble de commande respectif dudit appareil pour la chirurgie robotique, et au moins un premier pignon conique (11) associé à ladite poulie supplémentaire (10) pour transmettre les mouvements communiqués par les câbles de commande supplémentaires auxdits moyens d'effecteur (27).

8. Instrument pour la chirurgie robotique selon la revendication 7, **caractérisé en ce que** lesdits moyens d'effecteur (27) comprennent au moins une mâchoire (29) qui est montée en rotation sur l'un desdits bras transversaux (21 ; 210) pour tourner autour dudit second axe (Y) et comprend un second pignon conique (33) respectif s'engrenant avec ledit premier pignon conique (11) desdits éléments de pignon conique (9).

9. Instrument pour la chirurgie robotique selon la revendication 8, **caractérisé en ce que** ledit premier pignon conique (11) comprend une première surface externe tronconique ayant une première partie lisse (12) et une première partie dentée (13), qui est complémentaire de la première partie lisse (12) et ledit second pignon conique (33) comprend une seconde surface externe tronconique ayant une seconde partie lisse (34) et une seconde partie dentée (35) qui est complémentaire de la seconde partie lisse (34) ; le premier pignon conique (11) étant placé avec la première partie dentée (13) s'engrenant avec la seconde partie dentée (35) dudit second pignon conique (33).

10. Instrument pour la chirurgie robotique selon la revendication 5, **caractérisé en ce que** lesdits moyens de transmission (6) comprennent deux éléments de pignon conique (9), dont chacun est monté en rotation sur un bras respectif desdits bras axiaux (20 ; 200) pour tourner autour dudit premier axe (X), et lesdits moyens d'effecteur (27) comprennent deux mâchoires (29) dont chacune est montée en rotation sur un bras respectif desdits bras transversaux (21 ; 210) pour tourner autour dudit second axe (Y) et est couplé de manière cinématique uniquement à un élément respectif desdits éléments de pignon conique (9) pour permettre l'actionnement des deux mâchoires (29) indépendamment l'une de l'autre.

11. Instrument pour la chirurgie robotique selon la revendication 10, **caractérisé en ce que** chacun desdits éléments de pignon conique (9) comprend une poulie supplémentaire (10) respective qui est conçue pour être mise en prise par un câble de commande supplémentaire respectif dudit appareil pour la chirurgie robotique, et au moins un premier pignon conique (11) respectif associé à ladite poulie supplémentaire (10) ; chaque mâchoire (29) comprenant un second pignon conique (33) respectif qui s'engrène avec le premier pignon conique (11) de l'élément de pignon conique (9) associé à la mâchoire (29) pour transmettre les mouvements communiqués par le câble de commande supplémentaire respectif à la mâchoire (29) correspondante.

12. Instrument pour la chirurgie robotique selon la revendication 11, **caractérisé en ce que** chacun desdits premiers pignons coniques (11) comprend une première surface externe tronconique respective ayant une première partie lisse (12) et une première partie dentée (13) qui est complémentaire par rapport à la première partie lisse (12) et chacun desdits seconds pignons coniques (33) comprend une seconde surface externe tronconique respective ayant une seconde partie lisse (34) et une seconde partie dentée (35) qui est complémentaire par rapport à la seconde partie dentée (34) ; le premier pignon conique (11) de chaque élément de pignon conique (9) étant placé avec la première partie dentée (13) qui s'engrène avec la seconde partie dentée (35) du second pignon conique (33) d'une mâchoire (29) et avec la première partie lisse (12) faisant sensiblement face à la seconde partie lisse (34) du second pignon conique (33) de l'autre mâchoire (29) .

13. Instrument pour la chirurgie robotique selon la revendication 5, **caractérisé en ce que** chacun desdits bras axiaux et transversaux (20, 21) dudit élément de support et de déplacement (18 ; 180) comprend une partie de détection (22, 25) respective qui a au moins une partie de surface de plan parallèle ou orthogonale à un plan dans lequel lesdits premier et second axes (X, Y) se trouvent ; lesdits premiers moyens de capteur (28) étant placés sur au moins l'une des parties de surface de plan.

14. Instrument pour la chirurgie robotique selon la revendication 3, **caractérisé en ce que** lesdits moyens d'effecteur (27) comprennent au moins une mâchoire (29) qui est montée en rotation sur ledit élément de support et de déplacement (18, 180) et comprend un bras de support (31) et un élément terminal (32) ; lesdits seconds moyens de capteur (28) étant placés sur au moins un côté dudit bras de support (31).
